# EUROPEAN PATENT SPECIFICATION

(11) **EP 3 743 033 B1**
(45) Date of publication and mention of the grant of the patent: **25.03.2026**
(21) Application number: 19747863.9
(22) Date of filing: 31.01.2019
(51) Int. Cl.: A61G 13/00, A61G 7/00, A61G 7/10, A61G 13/10, A61G 7/002, A61G 7/005, A61G 13/12, A61F 5/37, A47C 20/02, A61G 1/044, A61G 1/01

(54) **BI-WING ARM SUPPORT SYSTEM**
ZWEIFLÜGELIGES ARMSTÜTZSYSTEM
SYSTÈME DE SUPPORT DE BRAS À DEUX AILES

(30) Priority: 31.01.2018 US 201862624357 P
(43) Date of publication of application: 02.12.2020
(73) Proprietor: Gomez, David J., Holly Springs, NC 27540 (US)
(72) Inventor: Gomez, David J., Holly Springs, NC 27540 (US)
(74) Representative: Forresters IP LLP
(86) International application number: PCT/US2019/016005
(87) International publication number: WO 2019/152624

(56) References cited:
- WO-A1-2017/184949
- WO-A1-2017/184949
- US-A1- 2015 297 435
- US-A1- 2015 297 435
- US-B1- 8 214 951
- US-B1- 8 601 623

## Description

### CROSS-REFERENCE TO RELATED APPLICATION

The present application claims priority benefit of U.S. Provisional Patent Application No. 62/624,357 filed on January 31, 2018, and entitled "Bi-Wing Arm Support System".

### FIELD OF THE INVENTION

The present invention relates generally to the field of medical devices for use in the surgical theater, and more particularly to an apparatus and method for securing a patient on a surgical table and for moving a patient from one position to another position on the surgical table.

### BACKGROUND ART

Routine movement and methodologies for moving patients on pad based systems for surgical tables begin to fail, especially during the care of moderate to morbidly obese patients. Rising obesity rates in the United States also place significant ergonomic safety and injury risk to healthcare providers as they must routinely physically lift and move these patients in order to facilitate surgical procedures. The healthcare industry faces a problem with a lack of products focused on improving methodologies and practices that facilitate less variance and safe care strategies, i.e., process improvements required for surgical positioning. The industry also lacks products designed to provide safer and more ergonomic processes for moving patients, which can be a key contributor to musculoskeletal disorders (MSD) and injuries (especially involving the back) suffered by providers.

US 8214951 discloses a soft surgical arm support and discloses the features defined in the preamble of claim 1. WO 2017/184949 discloses a patient positioning device. US 2015/297435 discloses a patient stabilization device and methods of use.

### BRIEF SUMMARY OF THE INVENTION

The present invention meets the above described need by providing an arm support apparatus (305) for use with a surgical table (15) as defined in claim 1.

### BRIEF DESCRIPTION OF THE DRAWINGS

Embodiments falling under the claimed invention are described in figures 1-3 and 12-16. The embodiments in the remaining figures are not according to the invention and present for background information only.
FIG. 1 is an exploded perspective of a bi-wing arm support apparatus according to the present invention.
FIG. 2 is a top plan view of the upper member of the apparatus shown in FIG. 1.
FIG. 3 is a top plan view of the lower member of the apparatus shown in FIG. 1.
FIG. 4 is a top plan view of one embodiment of the base of the apparatus.
FIG. 5 is a top plan view of the base of FIG. 4 supported on a surgical table.
FIG. 6 is a bottom plan view of a pad for use with the base of FIG. 4.
FIG. 7 is an exploded perspective view of the base and pad of FIGS. 4-6 in position on the surgical table.
FIG. 8 is a top perspective view of an alternate embodiment of the base.
FIG. 9A is a bottom perspective view of the base shown in FIG. 8.
FIG. 9B is an alternate embodiment of the bottom surface of the base.
FIG. 10 is a schematic view of another alternate embodiment of the base.
FIG. 11 is a perspective view of a subassembly of the pad system shown in FIG. 10.
FIG. 12 is a perspective view showing use of the bi-wing arm support apparatus on a patient.
FIG. 13 is a perspective view showing one of the initial stages of placement of the arm of the patient according to the present invention.
FIG. 14 is a perspective view showing the bi-wing arm support apparatus of the present invention partially positioned on a patient.
FIG. 15 is a perspective view showing one of the steps in positioning the arm of a patient by means of the present invention.
FIG. 16 is a perspective view showing the final step according to the present invention.
FIG. 17 is a perspective view of a pad used on the chest of the patient.

### DESCRIPTION OF THE PREFERRED EMBODIMENTS

At the outset, it should be clearly understood that like reference numerals are intended to identify the same structural elements, portions or surfaces consistently throughout the several drawing figures, as such elements, portions or surfaces may be further described or explained by the entire written specification, of which this detailed description is an integral part. Unless otherwise indicated, the drawings are intended to be read (*e.g.*, cross-hatching, arrangement of parts, proportion, debris, etc.) together with the specification, and are to be considered a portion of the entire written description of this invention. As used in the following description, the terms "horizontal", "vertical", "left", "right", "up" and "down", as well as adjectival and adverbial derivatives thereof, (e.g., "horizontally", "rightwardly", "upwardly", etc.), simply refer to the orientation of the illustrated structure as the particular drawing figure faces the reader. Similarly, the terms "inwardly" and "outwardly" generally refer to the orientation of a surface relative to its axis of elongation, or of rotation, as appropriate.

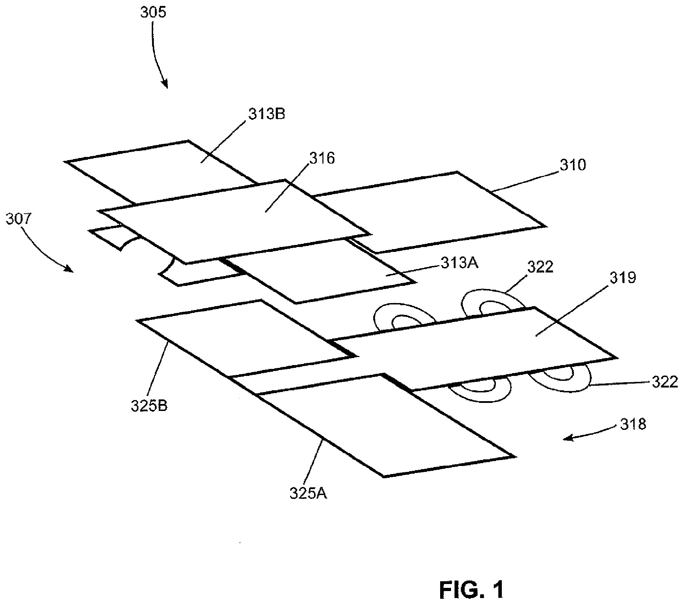

Referring now to the drawings, and more particularly to FIG. 1 thereof, this invention provides a bi-wing arm support apparatus 305. The bi-wing arm support apparatus 305 of the present invention includes an upper member 307 and a lower member 318. Upper member 307 includes a pad that may be divided into two sections 310 and 316. The pad may be constructed from foam or other resilient, elastic and/or cushioned materials. The pad may provide instant recovery of its original shape when the force is removed and may rely on friction to retain the patient in position on an angled surgical table. Alternatively, the pad may also be constructed of a viscoelastic material with slow recovery properties or of other viscoelastic materials having different recovery properties. Extending from opposite sides of pad section 316 are a pair of wing sections 3 13A and 313B constructed of a flexible material. The upper member 307 rests on top of the lower member 318. Lower member 318 includes a pad 319 that may include a main body portion. The pad may be constructed from foam or other resilient, elastic and/or cushioned materials. The pad may provide instant recovery of its original shape when the force is removed and may rely on friction to retain the patient in position on an angled surgical table. Alternatively, the pad may also be constructed of a viscoelastic material with slow recovery properties or of other viscoelastic materials having different recovery properties. One or more straps 322 may be provided for lifting and moving a patient supported by the lower member 318. A pair of wing sections 325A and 325B extend in opposite directions from the main body portion of pad 319.

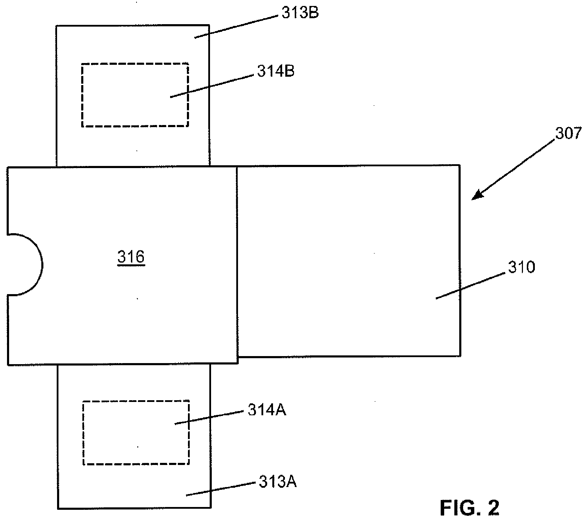

Turning to FIG. 2, a detailed view of the upper member 307 shows the positioning (in dashed lines) for a pair of hook and loop fastener sections 314A and 314B located on the underside of wing sections 313A and 313B.

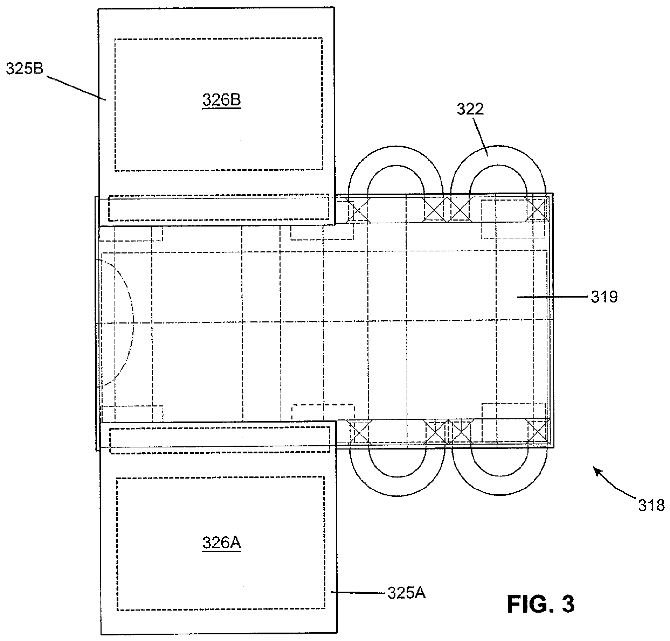

In FIG. 3, wings 325A and 325B may comprise disposable foam pad inserts that are attached to the main body of pad 319. The wing sections 325A and 325B may be provided with hook and loop fastening materials 326A and 326B on the underside as indicated by the dashed lines. The main body of the pad 319 may be constructed according to the disclosure in U.S. Patent Application No. 15/437,017 filed on February 20, 2017, and entitled "Apparatus for Securing a Patient in the Trendelenburg Position During Surgery". The lower member 318 is supported by a table such as an operating table in a surgical theater with the wings 325A and 325B extending from opposite sides. The upper member 307 is placed on top of the lower member 318 with wings 313A and 313B extending to the sides as shown in FIGS. 1 and 2.

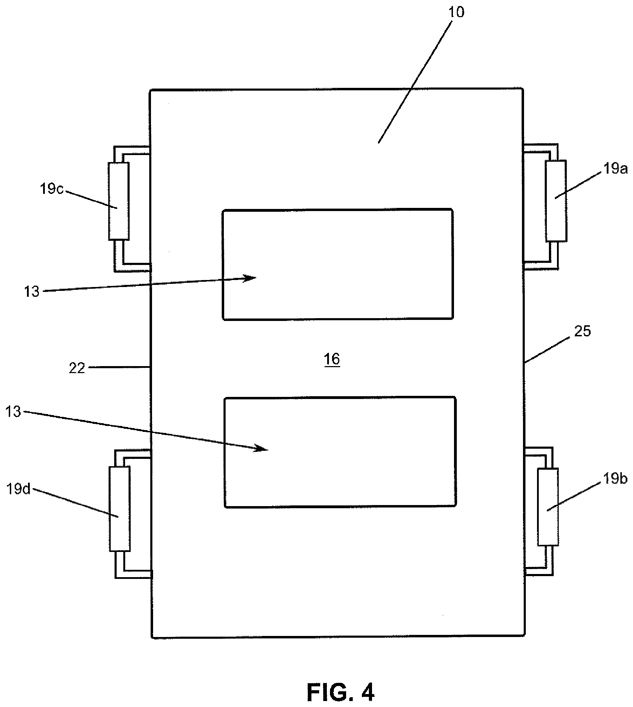

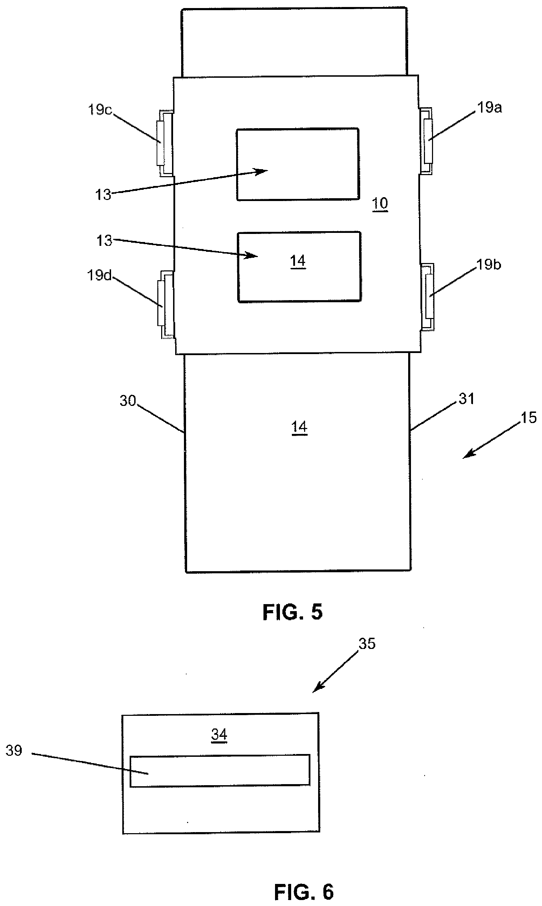

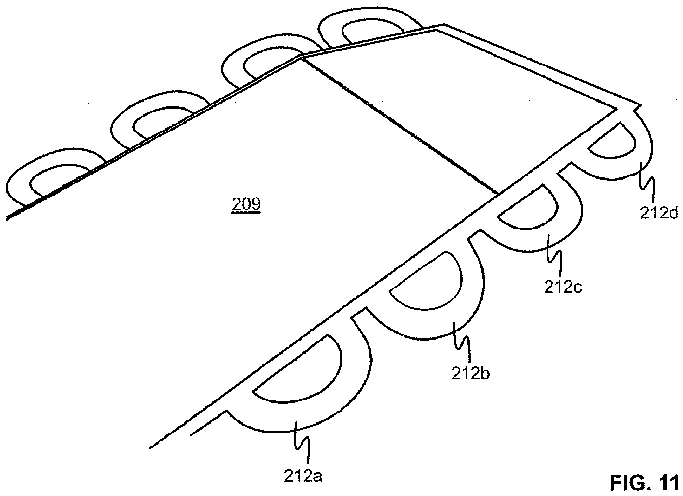

Referring to FIGS. 4-11, the main body of the pad 319 may be constructed as follows. Referring now to the drawings, and more particularly to FIG. 4 thereof, this embodiment provides a base 10 made of a flexible material. The base 10 may be provided with a least one opening 13 in a central portion 16. Turning to FIG. 5, the opening 13 exposes the top surface 14 of the surgical table 15 or surgical mattress through the base 10. Returning to FIG. 4, the base 10 is also provided with a plurality of handles 19a, 19b, 19c, and 19d disposed along the sides 22, 25 of the base 10. The handles 19a-d may be formed by loops of material attached to the sides 22, 25 of the base 10. The handles 19a-d are sized to receive the hand of a provider such that the provider can grasp the handles 19a-d with their hands in order to move the base 10 along the length of the surgical table 15 to position and re-position the patient as necessary during a surgical procedure.

Turning to FIG. 5, the base 10 is shown in position relative to a surgical table 15. As shown the handle portions 19a-d of the base 10 extend along the sides 30, 31 of the surgical table 15 and are in a position to be grasped by a provider standing next to the surgical table 15. In this manner, a provider positioned on each side of the surgical table 15 can move the patient easily in either direction along the surgical table 15 by grabbing the handles 19a-d and lifting the patient with the cooperation of a provider standing on the opposite side. Alternatively, two providers on each side can each grab a handle 19a-d to move heavier patients.

In FIG. 6, the underside 34 of a pad 35 is shown. The underside 34 has a pressure sensitive adhesive strip 39 disposed thereon. The pressure sensitive adhesive strip 39 may be provided with a protective cover (not shown) that is removed prior to use. The cover is removed from the pressure sensitive adhesive strip 39 and then the pad 35 is placed over the base 10 such that the pressure sensitive adhesive strip 39 aligns with the openings 13 in the base 10. The pressure sensitive adhesive strip 39 makes contact with the top surface 14 of the surgical table 15 through the openings 13 in the base 10. As a result, the pad 35 is held in position on the surgical table 15 by means of the pressure sensitive adhesive strip 39. The adhesive strip 39 prevents the pad 35 and base 10 from sliding relative to the surgical table 15, but can be removed for repositioning the pad 35 on the surgical table 15. When the pressure sensitive adhesive strip 39 on the bottom 34 of the pad 35 is lifted off of the surgical table 15, the adhesive strip 39 does not leave any residue on the surface of the surgical table 15.

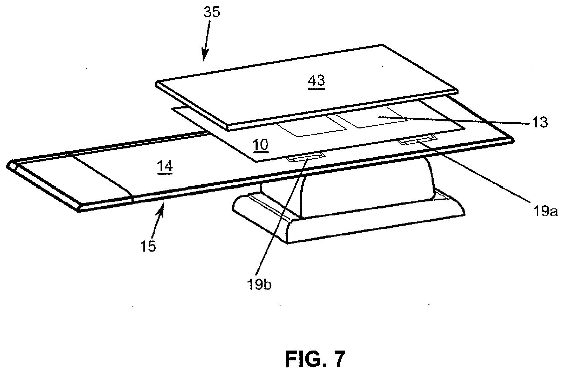

In FIG. 7, the base 10 is placed directly onto the surgical table 15 and can be repositioned by means of the handles 19a-d extending from the sides 22, 25 of the base 10. The pad 35 having a re-positionable pressure sensitive adhesive strip 39 on a bottom surface 34 (FIG. 3) is placed on top of the base 10 such that the pressure sensitive adhesive strip 39 aligns with the openings 13 in the base 10. Next, the patient is placed on the top surface 43 of the pad 35.

It is very common for patients to be routinely moved after anesthesia induction and laryngoscopy (endotracheal tube placement), to another position on the surgical table (pad or gel) in order to facilitate surgical care. More routinely is the need to move the patient in a range of 5.08 cm to 30.48 cm (2 to 12 inches) distally towards the foot of the bed to facilitate positions like lithotomy. It is also very common for anesthesia providers to request the patient at the head of the surgical table, proximal to the provider. This is a safety concern as securing of the airway is one of the most important aspects of anesthesia care. In patients of moderate to morbid obesity, it is important to facilitate this approximation to the provider for many clinical and safety reasons.

The very nature of the padding used in standard surgical practice is to reduce potential for pressure related injuries to skin, muscle and more importantly nerves. This is the nature of padding in the perioperative setting. The apparatus of the present disclosure supports the notion of optimizing safety for the patient and the staff through its multiple and cumulative utility applications. Understanding routine processes of care in relation to how patients are positioned within the operating room along with staff methodologies is key to understanding the intent of the present design and its use within the perioperative setting.

The apparatus of the present disclosure provides an approach to moving patients during routine and well known surgical procedures requiring positioning changes while securing them via a unique material (nonwoven and durable re-usable) reinforced pad using a non-residue pressure sensitive adhesive. The apparatus of the present disclosure reduces the need to pick the patient off a viscoelastic/gel-based pad via either "draw sheet" or awkward manual movements conducted by staff, providing for a fully moveable system that moves both the pad and the patient.

The apparatus of the present disclosure provides an improved disposable, nonwoven fused pad with a pressure sensitive adhesive for holding the pad in place on the surgical table in place of hook and loop fastener straps attached to the bed rail. The disposable foam pad works in conjunction with the reusable patient lifting/moving non durable material base, allowing nurses to more readily and easily move patients on the table in order to facilitate surgical positioning. The pressure sensitive adhesive holds the pad in place and prevents slippage between the pad and the top of the surgical table. Furthermore, the pressure sensitive adhesive will not leave an adhesive residue which provides for better cleaning and decontamination. Historically, it is known that adhesive residue is a haven for opportunistic infectious pathogens within the clinical setting. Many pathogens reside in residue, potentially leading to hospital acquired infections (HAI's) and/or surgical site infections (SSI's).

The pad of the present disclosure will remain in contact with the surgical mattress through its durable material base, yet allows for movement of the entire apparatus when used to move a patient. The pressure sensitive adhesive on the bottom of the pad releases contact when the patient is lifted via the straps on the base and then resettles and reattaches to the surgical mattress when the patient is moved to their final surgical table position which keeps the entire apparatus in place during gravity dependent surgical table positions commonly used during surgery. This is not possible with current systems that require hook and loop fastener based straps. There is also not enough support structure to allow standard viscoelastic pads to lift and move patients. The ergonomic handles 19a-d on the base 10 improve and optimize ergonomic methods for providers during patient repositioning and improve the efficiency and efficacy of handling the patient.

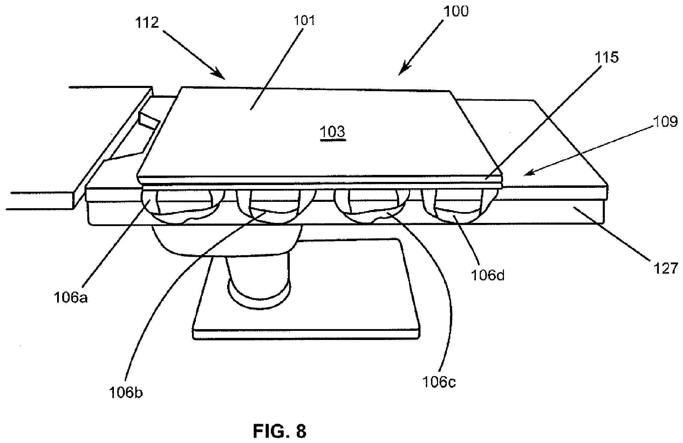

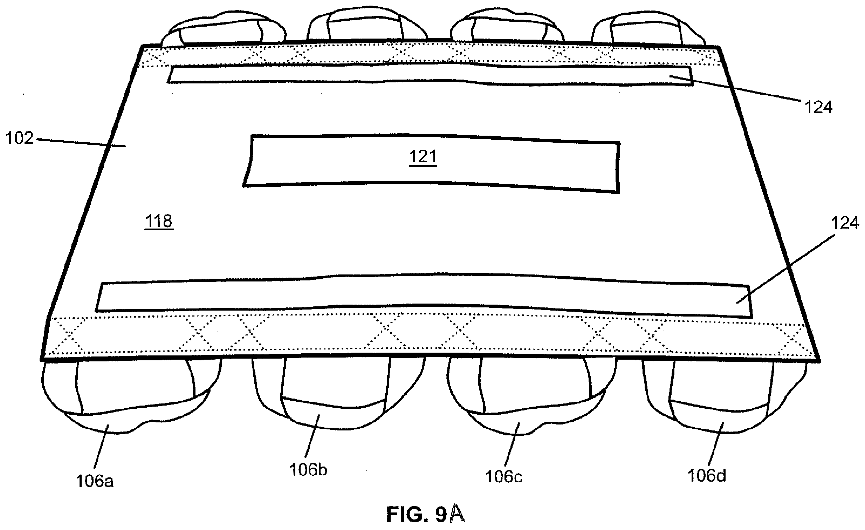

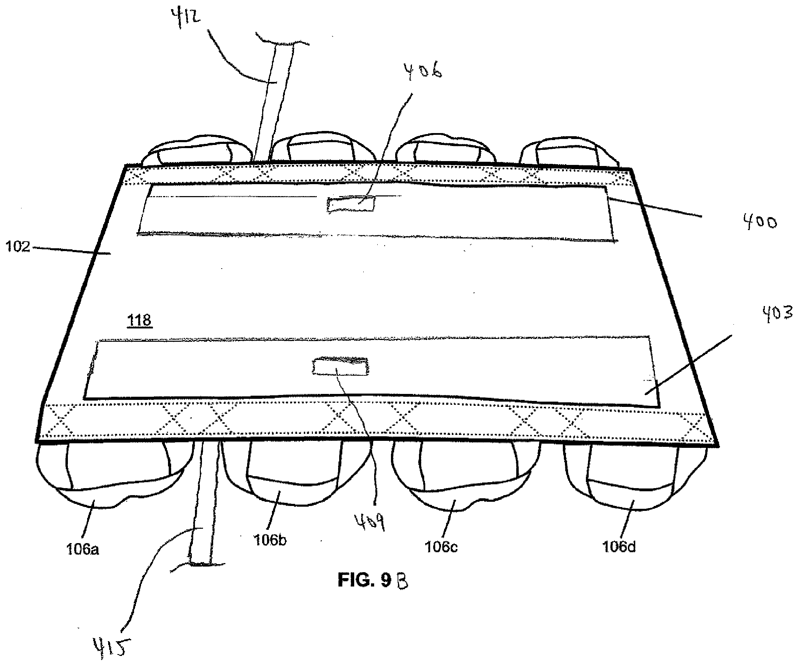

Turning to FIG. 8 in another embodiment, a disposable pad assembly 100 may be formed by fusing a standard use pad 101 to a nonwoven layered material 102 (FIG. 9) such as SMS (spunbond-meltblown-spunbond) or similar celluloid material. The pad assembly 100 may be provided with multiple handles 106a-d along opposed outside edges 109, 112 to provide grabbing points for lifting the pad assembly 100 and the patient (not shown) during use. In use, the patient is positioned on surface 103. The pad assembly 100 may also include one or more additional reinforcement layers 115 constructed of a condensed foam or biodegradable cardboard piece (placed between the nonwoven layers of the pad assembly 100). This reinforced pad assembly structure 115 keeps the whole pad assembly 100 from bunching up under the patient when lifting and/or repositioning the patient. It also allows the provider to pull back in case the patient was not positioned correctly. This adjustment is to facilitate distal perineal placement of the patient distally toward the surgical field. Currently the patients have to be repeatedly moved toward the distal end of the pad to keep the perineal space open for surgery. The nonwoven is separated from the pad at this end, allowing the nonwoven to be folded under thereby improving perineal placement. A warming support structure inlay may be placed between nonwoven materials in place of the foam or cardboard support structure 115. This warming support structure allows the transfer of heat to the patient from under the pad system, reinforcing the foams natural ability to be both insulator and conductor of heat. The bottom surface 118 (FIG. 9A) of the pad assembly 100 may be provided with an anti-skid rubber foam strip 121 and/or a pressure sensitive adhesive (PSA) strip 124. Alternatively, as shown in FIG. 9B the bottom surface 118 may be provided with two strips 400, 403 of foam for traction plus pressure sensitive adhesive sections 406, 409. The center of the surface 118 may be left open without any foam or pressure sensitive adhesive disposed thereon. The pad assembly 100 may also include a tether 412, 415 on each side for attaching the apparatus to the rail of a surgical table. This pad assembly 100 allows patients to be lifted evenly as weight is distributed through the semi-rigid support structure 115 under the pad 101. After use, the entire pad assembly 100 may be discarded. In FIG. 8, the pad assembly 100 is shown supported by a surgical table 127.

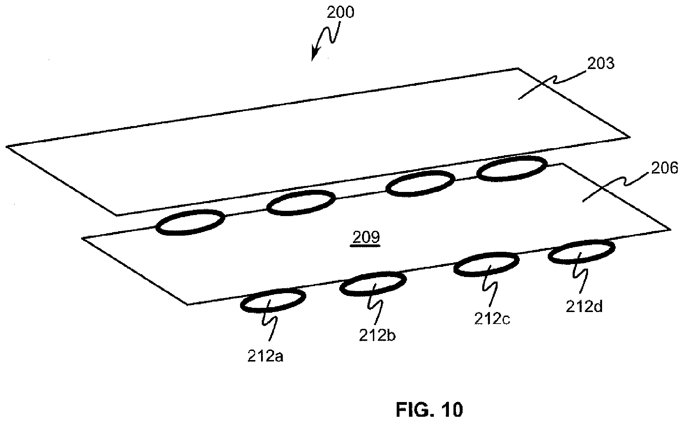

In FIG. 10, in another embodiment, a non-disposable pad assembly 200 may be constructed of two major components: a disposable pad subassembly 203 and a lower support structure 206 that may be cleaned for repeated use. The disposable pad subassembly 203 may be constructed of a standard foam pad that may be fused with an upper support structure. The upper support structure may be provided with an FDA approved adhesive or a similar bonding strength pressure sensitive adhesive (PSA) on its bottom surface for removably attaching the pad subassembly 203 to the lower support structure 206.

The lower support structure 206 has an upper surface 209 and a lower surface (not shown). The lower support structure 206 receives the pad subassembly 203 on the upper surface 209 and the lower surface of the lower support structure 206 rests on the surgical table 127 (FIG. 8). The lower surface may be provided with an anti-skid rubber foam strip and a pressure sensitive adhesive (PSA) strip for holding the pad subassembly in position on the surgical table 127. The lower support structure 206 may be provided with multiple handles 212a-d that provide a grabbing point for lifting the patient for transfer from one position to another position on the surgical table.

The lower support structure 206 may be formed from an FDA approved and cleanable woven fabric material. The internal structure of the disposable pad subassembly 203 may include both viscoelastic and a compressible/expandable foam fused with a dense and compressible foam support structure. This combination prevents sagging or bunching of the materials with respect to the patient during a lifting scenario. The lower structure 206 may be enclosed via a cleanable fabric. As described above a heating element may be incorporated into the design which allows the device to be a modular patient warming and positioning device. Current under body warming mattress top systems are not modular, do not contain handles, and force providers to place the patient in a less than optimal position.

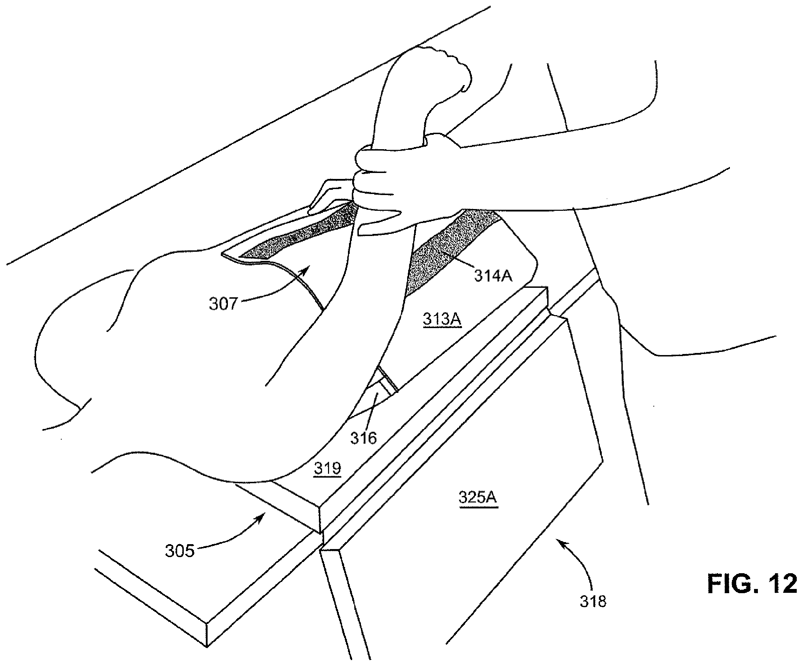

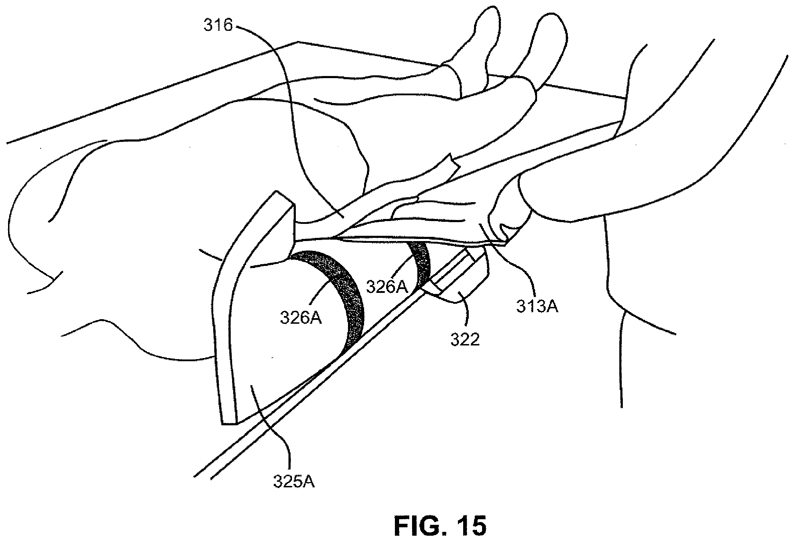

Turning to FIGS. 12-16, the bi-wing arm support apparatus 305 of the present invention is used as follows. A patient lying on their back is positioned on top of the upper member 307 which in turn rests on the lower member 318 to form the bi-wing arm support apparatus 305 of the present invention. The arm of the patient is lifted as shown in FIG. 12 and the wing 313A is folded upward where it rests on the body of the patient and exposes the hook and loop fasteners 314A on the underside of the wing 313A. The patient's arm is placed on the pad 319 of the lower member 318 where it rests during the procedure.

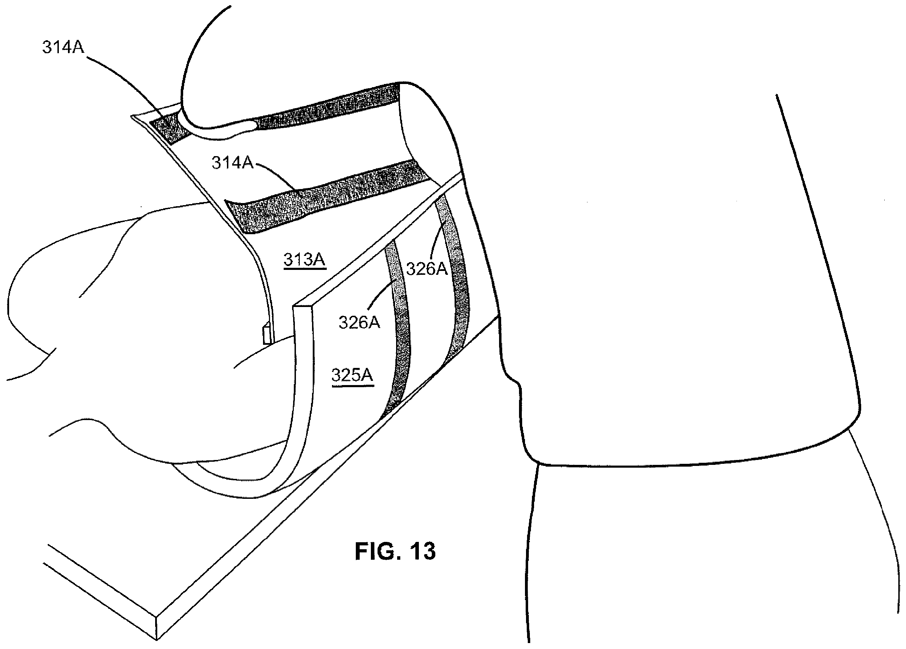

In FIG. 13, the wing 325A is rotated upward around the arm. In the position shown hook and loop fastening surfaces 326A on the underside of the wing 325A are exposed when the wing 325A is rotated upward.

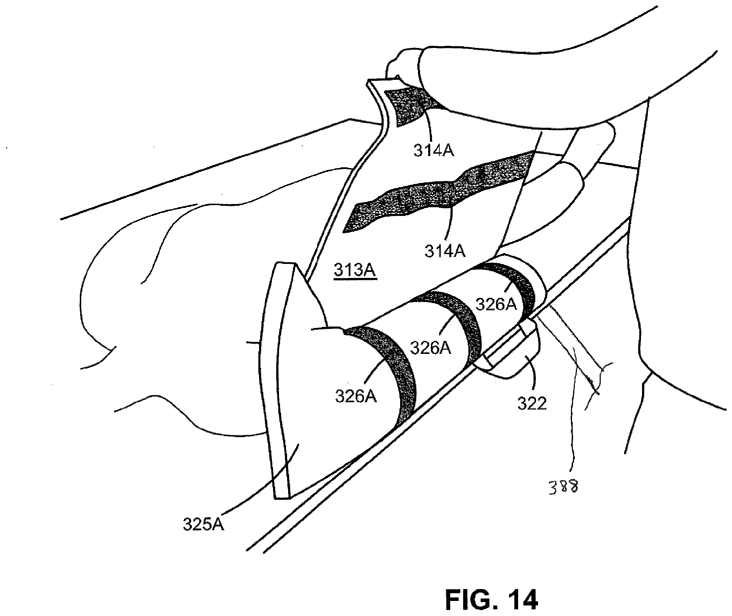

Turning to FIGS. 14-15, with the wing 325A wrapped around the arm, the wing 313A of the upper member 307 is rotated downward around the arm such that the hook and loop fasteners 314A on the bottom of the wing 313A engage with the hook and loop fasteners 326A on the exposed bottom surface of wing 325A. The apparatus 305 may also be provided with a tether 388 on each side. The tether 388 may be constructed of a nonwoven material having cooperating hook and loop fastening material surfaces for attaching the tether 388 to the rail on a surgical table.

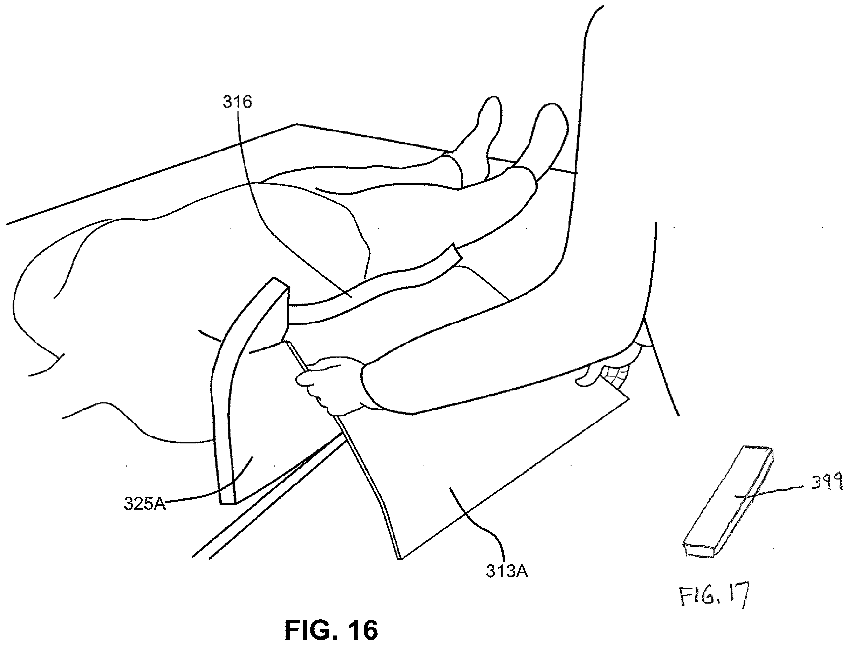

As shown in Fig. 16, the arm is completely wrapped and the wings 313A and 325A are attached by the hook and loop fasteners. In this position the arm has been securely wrapped without use of the patient's body weight, and the arm can be easily unwrapped by reversing the steps above, without the necessity of repositioning the patient (to get their body weight off of the device or a draw sheet), for access to the arm during the surgical procedure. Access may be necessary for anesthesia monitoring lines, invasive arterial lines, non-invasive blood pressure cuffs, SPO2 finger probes, and intravenous lines needed for medications and fluid delivery.

In FIG. 17, a pad 399 for wrapping around the chest of a patient is shown. The pad 399 may be constructed from a rectangular shaped piece of foam and may be secured across the chest of the patient by a strap.

The present invention contemplates that many changes and modifications may be made. Therefore, while the presently-preferred form of the bi-wing arm support system has been shown and described, and several modifications and alternatives discussed, persons skilled in this art will readily appreciate that various additional changes and modifications may be made without departing from the invention.

## Claims

1. An arm support apparatus (305) for use with a surgical table (15), the arm support apparatus (305), comprising:
an upper member (307) having an elongate pad, with a first side, a second side, a top surface and a bottom surface;
a first flexible wing (313A) configured to be rotated downward around the arm and extending from the first side of the upper member (307), the first flexible wing (313A) having a top surface and a bottom surface, one of a hook and loop fastening material (314A) disposed on the bottom surface of the first flexible wing (313A);
a second flexible wing (313B) configured to be rotated downward around the arm and extending from the second side of the upper member (307), the second flexible wing (313B) having a top surface and a bottom surface, one of a hook and loop fastening material (314B) disposed on the bottom surface of the second flexible wing (313B);
a lower member (318) having an elongate pad (319), with a first side, a second side, a top surface and a bottom surface, the bottom surface of the lower member (318) for facing the top surface (14) of the surgical table (15) and the upper member (307) configured to rest on the lower member (318);
a third flexible wing (325A) configured to be wrapped around the arm and extending from the first side of the lower member (318), the third flexible wing (325A) having a top surface and a bottom surface, one of a hook and loop fastening material (326A) disposed on the bottom surface of the third flexible wing (325A) and configured to engage with the hook and loop fastening material (314A) of the first flexible wing (313A); and,
a fourth flexible wing (325B) extending from the second side of the lower member (318), the fourth flexible wing (325B) having a top surface and a bottom surface, one of a hook and loop fastening material (326B) disposed on the bottom surface of the fourth flexible wing (325B) and configured to engage with the hook and loop fastening material (314B) of the second flexible wing (313B), **characterized by**
the lower member (318) having a plurality of handles (322) extending from the first and second sides of the lower member (318), wherein the bottom surface of the lower member (318) has a strip of material disposed thereon for inhibiting movement of the bottom surface relative to the surgical table (15), wherein the strip of material further comprises a pressure sensitive adhesive (39).

2. The arm support apparatus (305) of Claim 1, wherein the lower member (318) further comprises a nonwoven material.

3. The arm support apparatus (305) of Claim 1, wherein the lower member (318) further comprises a spunbond-meltblown-spunbond (SMS) material.

4. The arm support apparatus (305) of Claim 1, wherein the lower member (318) further comprises a celluloid material.

5. The arm support apparatus (305) of Claim 1, further comprising a warming support structure inlay.

6. The arm support apparatus (305) of Claim 1, wherein the lower member (318) further comprises a reinforcing layer.

7. The arm support apparatus (305) of Claim 6, wherein the reinforcing layer is foam.

8. The arm support apparatus (305) of Claim 6, wherein the reinforcing layer is cardboard.

9. A method for supporting the arm of a patient on a surgical table (15), the method, comprising:
Providing an upper member (307) having an elongate pad, with a first side, a second side, a top surface and a bottom surface;
providing a first flexible wing (313A) extending from the first side of the upper member (307), the first flexible wing (313A) having a top surface and a bottom surface, one of a hook and loop fastening material (314A) disposed on the bottom surface of the first flexible wing (313A);
providing a second flexible wing (313B) extending from the second side of the upper member (307), the second flexible wing (313B) having a top surface and a bottom surface, one of a hook and loop fastening material (314B) disposed on the bottom surface of the second flexible wing;
providing a lower member (318) having an elongate pad (319), with a first side, a second side, a top surface and a bottom surface, the bottom surface of the lower member (318) facing the top surface (14) of the surgical table (15), the lower member (318) having a plurality of handles (322) extending from the first and second sides of the lower member (318), wherein the bottom surface of the lower member (318) has a strip of material disposed thereon for inhibiting movement of the bottom surface relative to the surgical table (15), wherein the strip of material further comprises a pressure sensitive adhesive (39);
providing a third flexible wing (325A) extending from the first side of the lower member (318), the third flexible wing (325A) having a top surface and a bottom surface, one of a hook and loop fastening material (326A) disposed on the bottom surface of the third flexible wing (325A);
providing a fourth flexible wing (325B) extending from the second side of the lower member (318), the fourth flexible wing (325B) having a top surface and a bottom surface, one of a hook and loop fastening material (326B) disposed on the bottom surface of the fourth flexible wing (325B);
lifting the arm of the patient;
folding the first flexible wing (313A) of the upper member (307) upward and resting it on the patient's body;
placing the patient's arm on the pad (319) of the lower member (318);
rotating the third flexible wing (325A) on the lower member (318) upward around the arm of the patient;
rotating the first flexible wing (313A) on the upper member (307) downward around the arm such that the hook and loop fastening material (314A) on the bottom surface of the first flexible wing (313A) on the upper member (307) engages with the hook and loop fastening material (326A) on the bottom surface of the third flexible wing (325A) on the lower member (318).

## Patentansprüche

1. Armstützeinrichtung (305) zur Verwendung mit einem Operationstisch (15), die Armstützeinrichtung (305) umfassend:
ein oberes Element (307) aufweisend ein längliches Polster, mit einer ersten Seite, einer zweiten Seite, einer oberen Oberfläche und einer unteren Oberfläche;
einen ersten flexiblen Flügel (313A), der konfiguriert ist, um um den Arm nach unten gedreht zu werden, und der sich von der ersten Seite des oberen Elements (307) erstreckt, wobei der erste flexible Flügel (313A) eine obere Oberfläche und eine untere Oberfläche aufweist, wobei eines von einem Klettverschlussmaterial (314A) auf der unteren Oberfläche des ersten flexiblen Flügels (313A) angeordnet ist;
einen zweiten flexiblen Flügel (313B), der konfiguriert ist, um um den Arm nach unten gedreht zu werden, und der sich von der zweiten Seite des oberen Elements (307) erstreckt, wobei der zweite flexible Flügel (313B) eine obere Oberfläche und eine untere Oberfläche aufweist, wobei eines von einem Klettverschlussmaterial (314B) auf der unteren Oberfläche des zweiten flexiblen Flügels (313B) angeordnet ist;
ein unteres Element (318) aufweisend ein längliches Polster (319), mit einer ersten Seite, einer zweiten Seite, einer oberen Oberfläche und einer unteren Oberfläche, wobei die untere Oberfläche des unteren Elements (318) der oberen Oberfläche (14) des Operationstisches (15) zugewandt ist und das obere Element (307) konfiguriert ist, um auf dem unteren Element (318) aufzuliegen;
einen dritten flexiblen Flügel (325A), der konfiguriert ist, um um den Arm gewickelt zu werden, und der sich von der ersten Seite des unteren Elements (318) erstreckt, wobei der dritte flexible Flügel (325A) eine obere Oberfläche und eine untere Oberfläche aufweist, eines von einem Klettverschlussmaterial (326A), das auf der unteren Oberfläche des dritten flexiblen Flügels (325A) angeordnet und konfiguriert ist, um mit dem Klettverschlussmaterial (314A) des ersten flexiblen Flügels (313A) in Eingriff zu treten; und
einen vierten flexiblen Flügel (325B), der sich von der zweiten Seite des unteren Elements (318) erstreckt, wobei der vierte flexible Flügel (325B) eine obere Oberfläche und eine untere Oberfläche aufweist, eines von einem Klettverschlussmaterial (326B), das auf der unteren Oberfläche des vierten flexiblen Flügels (325B) angeordnet und konfiguriert ist, um mit dem Klettverschlussmaterial (314B) des zweiten flexiblen Flügels (313B) in Eingriff zu treten, **gekennzeichnet durch** das untere Element (318), das eine Vielzahl von Griffen (322) aufweist, die sich von der ersten und zweiten Seite des unteren Elements (318) erstrecken, wobei die untere Oberfläche des unteren Elements (318) einen darauf angeordneten Materialstreifen zum Hemmen der Bewegung der unteren Oberfläche relativ zum Operationstisch (15) aufweist, wobei der Materialstreifen ferner einen druckempfindlichen Klebstoff (39) umfasst.

2. Armstützeinrichtung (305) nach Anspruch 1, wobei das untere Element (318) ferner ein Vliesstoffmaterial umfasst.

3. Armstützeinrichtung (305) nach Anspruch 1, wobei das untere Element (318) ferner ein Spunbond-Meltblown-Spunbond-Material (SMS-Material) umfasst.

4. Armstützeinrichtung (305) nach Anspruch 1, wobei das untere Element (318) ferner ein Zelluloidmaterial umfasst.

5. Armstützeinrichtung (305) nach Anspruch 1, ferner umfassend eine wärmende Stützkörpereinlage.

6. Armstützeinrichtung (305) nach Anspruch 1, wobei das untere Element (318) ferner eine Verstärkungsschicht umfasst.

7. Armstützeinrichtung (305) nach Anspruch 6, wobei die Verstärkungsschicht Schaum ist.

8. Armstützeinrichtung (305) nach Anspruch 6, wobei die Verstärkungsschicht Pappe ist.

9. Verfahren zum Stützen des Arms eines Patienten auf einem Operationstisch (15), das Verfahren umfassend:
Bereitstellen eines oberen Elements (307) mit einem länglichen Polster, mit einer ersten Seite, einer zweiten Seite, einer oberen Oberfläche und einer unteren Oberfläche;
Bereitstellen eines ersten flexiblen Flügels (313A), der sich von der ersten Seite des oberen Elements (307) erstreckt, wobei der erste flexible Flügel (313A) eine obere Oberfläche und eine untere Oberfläche aufweist, wobei eines von einem Klettverschlussmaterial (314A) auf der unteren Oberfläche des ersten flexiblen Flügels (313A) angeordnet ist;
Bereitstellen eines zweiten flexiblen Flügels (313B), der sich von der zweiten Seite des oberen Elements (307) erstreckt, wobei der zweite flexible Flügel (313B) eine obere Oberfläche und eine untere Oberfläche aufweist, wobei eines von einem Klettverschlussmaterial (314B) auf der unteren Oberfläche des zweiten flexiblen Flügels angeordnet ist;
Bereitstellen eines unteren Elements (318) mit einem länglichen Polster (319), mit einer ersten Seite, einer zweiten Seite, einer oberen Oberfläche und einer unteren Oberfläche, wobei die untere Oberfläche des unteren Elements (318) der oberen Oberfläche (14) des Operationstisches (15) zugewandt ist, wobei das untere Element (318) eine Vielzahl von Griffen (322) umfasst, die sich von der ersten und zweiten Seite des unteren Elements (318) erstrecken, wobei die untere Oberfläche des unteren Elements (318) einen darauf angeordneten Materialstreifen zum Hemmen der Bewegung der unteren Oberfläche relativ zum Operationstisch (15) aufweist, wobei der Materialstreifen ferner einen druckempfindlichen Klebstoff (39) umfasst;
Bereitstellen eines dritten flexiblen Flügels (325A), der sich von der ersten Seite des unteren Elements (318) erstreckt, wobei der dritte flexible Flügel (325A) eine obere Oberfläche und eine untere Oberfläche aufweist, wobei eines von einem Klettverschlussmaterial (326A) auf der unteren Oberfläche des dritten flexiblen Flügels (325A) angeordnet ist;
Bereitstellen eines vierten flexiblen Flügels (325B), der sich von der zweiten Seite des unteren Elements (318) erstreckt, wobei der vierte flexible Flügel (325B) eine obere Oberfläche und eine untere Oberfläche aufweist, wobei eines von einem Klettverschlussmaterial (326B) auf der unteren Oberfläche des vierten flexiblen Flügels (325B) angeordnet ist;
Anheben des Arms des Patienten;
Falten des ersten flexiblen Flügels (313A) des oberen Elements (307) nach oben und Auflegen desselben auf den Körper des Patienten;
Platzieren des Arms des Patienten auf dem Polster (319) des unteren Elements (318);
Drehen des dritten flexiblen Flügels (325A) an dem unteren Element (318) nach oben um den Arm des Patienten;
Drehen des ersten flexiblen Flügels (313A) an dem oberen Element (307) nach unten um den Arm, derart, dass das Klettverschlussmaterial (314A) auf der unteren Oberfläche des ersten flexiblen Flügels (313A) an dem oberen Element (307) mit dem Klettverschlussmaterial (326A) auf der unteren Oberfläche des dritten flexiblen Flügels (325A) an dem unteren Element (318) in Eingriff kommt.

## Revendications

1. Appareil de support de bras (305) pour utilisation avec une table chirurgicale (15), l'appareil de support de bras (305), comprenant :
un élément supérieur (307) ayant un coussinet allongé, avec un premier côté, un second côté, une surface supérieure et une surface inférieure ;
une première ailette flexible (313A) conçue pour être mise en rotation vers le bas autour du bras et s'étendant à partir du premier côté de l'élément supérieur (307), la première ailette flexible (313A) ayant une surface supérieure et une surface inférieure, l'un parmi un matériau de fixation à crochets et à boucles (314A) étant disposé sur la surface inférieure de la première ailette flexible (313A) ;
une deuxième ailette flexible (313B) conçue pour être mise en rotation vers le bas autour du bras et s'étendant à partir du second côté de l'élément supérieur (307), la deuxième ailette flexible (313B) ayant une surface supérieure et une surface inférieure, l'un parmi un matériau de fixation à crochets et à boucles (314B) étant disposé sur la surface inférieure de la deuxième ailette flexible (313B) ;
un élément inférieur (318) ayant un coussinet allongé (319), avec un premier côté, un second côté, une surface supérieure et une surface inférieure, la surface inférieure de l'élément inférieur (318) étant destinée à faire face vers la surface supérieure (14) de la table chirurgicale (15) et l'élément supérieur (307) étant conçu pour reposer sur l'élément inférieur (318) ;
une troisième ailette flexible (325A) conçue pour être enveloppée autour du bras et s'étendant à partir du premier côté de l'élément inférieur (318), la troisième ailette flexible (325A) ayant une surface supérieure et une surface inférieure, l'un parmi un matériau de fixation à crochets et à boucles (326A) étant disposé sur la surface inférieure de la troisième ailette flexible (325A) et conçu pour venir en prise avec le matériau de fixation à crochets et à boucles (314A) de la première ailette flexible (313A) ; et,
une quatrième ailette flexible (325B) s'étendant à partir du second côté de l'élément inférieur (318), la quatrième ailette flexible (325B) ayant une surface supérieure et une surface inférieure, l'un parmi un matériau de fixation à crochets et à boucles (326B) étant disposé sur la surface inférieure de la quatrième ailette flexible (325B) et conçu pour venir en prise avec le matériau de fixation à crochets et à boucles (314B) de la deuxième ailette flexible (313B), **caractérisé par** l'élément inférieur (318) ayant une pluralité de poignées (322) s'étendant à partir des premier et second côtés de l'élément inférieur (318), dans lequel la surface inférieure de l'élément inférieur (318) a une bande de matériau disposée sur celle-ci permettant d'inhiber un mouvement de la surface inférieure par rapport à la table chirurgicale (15), dans lequel la bande de matériau comprend en outre un adhésif sensible à la pression (39).

2. Appareil de support de bras (305) selon la revendication 1, dans lequel l'élément inférieur (318) comprend en outre un matériau non tissé.

3. Appareil de support de bras (305) selon la revendication 1, dans lequel l'élément inférieur (318) comprend en outre un matériau filé-lié/soufflé en fusion/filé-lié (SMS).

4. Appareil de support de bras (305) selon la revendication 1, dans lequel l'élément inférieur (318) comprend en outre un matériau de celluloïd.

5. Appareil de support de bras (305) selon la revendication 1, comprenant en outre une insertion de structure de support chauffante.

6. Appareil de support de bras (305) selon la revendication 1, dans lequel l'élément inférieur (318) comprend en outre une couche de renfort.

7. Appareil de support de bras (305) selon la revendication 6, dans lequel la couche de renfort est de la mousse.

8. Appareil de support de bras (305) selon la revendication 6, dans lequel la couche de renfort est du carton.

9. Procédé permettant de supporter le bras d'un patient sur une table chirurgicale (15), le procédé, comprenant :
la fourniture d'un élément supérieur (307) ayant un coussinet allongé, avec un premier côté, un second côté, une surface supérieure et une surface inférieure ;
la fourniture d'une première ailette flexible (313A) s'étendant à partir du premier côté de l'élément supérieur (307), la première ailette flexible (313A) ayant une surface supérieure et une surface inférieure, l'un parmi un matériau de fixation à crochets et à boucles (314A) étant disposé sur la surface inférieure de la première ailette flexible (313A) ;
la fourniture d'une deuxième ailette flexible (313B) s'étendant à partir du second côté de l'élément supérieur (307), la deuxième ailette flexible (313B) ayant une surface supérieure et une surface inférieure, l'un parmi un matériau de fixation à crochets et à boucles (314B) étant disposé sur la surface inférieure de la deuxième ailette flexible ;
la fourniture d'un élément inférieur (318) ayant un coussinet allongé (319), avec un premier côté, un second côté, une surface supérieure et une surface inférieure, la surface inférieure de l'élément inférieur (318) faisant face vers la surface supérieure (14) de la table chirurgicale (15), l'élément inférieur (318) ayant une pluralité de poignées (322) s'étendant à partir des premier et second côtés de l'élément inférieur (318), dans lequel la surface inférieure de l'élément inférieur (318) a une bande de matériau disposée sur celle-ci permettant d'inhiber un mouvement de la surface inférieure par rapport à la table chirurgicale (15), dans lequel la bande de matériau comprend en outre un adhésif sensible à la pression (39) ;
la fourniture d'une troisième ailette flexible (325A) s'étendant à partir du premier côté de l'élément inférieur (318), la troisième ailette flexible (325A) ayant une surface supérieure et une surface inférieure, l'un parmi un matériau de fixation à crochets et à boucles (326A) étant disposé sur la surface inférieure de la troisième ailette flexible (325A) ;
la fourniture d'une quatrième ailette flexible (325B) s'étendant à partir du second côté de l'élément inférieur (318), la quatrième ailette flexible (325B) ayant une surface supérieure et une surface inférieure, l'un parmi un matériau de fixation à crochets et à boucles (326B) étant disposé sur la surface inférieure de la quatrième ailette flexible (325B) ;
le soulèvement du bras du patient ;
le pliage de la première ailette flexible (313A) de l'élément supérieur (307) vers le haut et le dépôt de celle-ci sur le corps du patient ;
la mise en place du bras du patient sur le coussinet (319) de l'élément inférieur (318) ;
la rotation de la troisième ailette flexible (325A) sur l'élément inférieur (318) vers le haut autour du bras du patient ;
la rotation de la première ailette flexible (313A) sur l'élément supérieur (307) vers le bas autour du bras de telle sorte que le matériau de fixation à crochets et à boucles (314A) sur la surface inférieure de la première ailette flexible (313A) sur l'élément supérieur (307) vient en prise avec le matériau de fixation à crochets et à boucles (326A) sur la surface inférieure de la troisième ailette flexible (325A) sur l'élément inférieur (318).
